# EUROPÄISCHE PATENTANMELDUNG

(11) **EP 3 029 402 A1**
(43) Veröffentlichungstag der Anmeldung: **08.06.2016**
(21) Anmeldenummer: 14196654.9
(22) Anmeldetag: 05.12.2014
(51) Int. Cl.: F25J 3/02

(54) **Verfahren und Anlage zur trenntechnischen Bearbeitung eines Einsatzstroms**

(71) Anmelder: Linde Aktiengesellschaft, 80331 München (DE)
(72) Erfinder: Pham Duc, Tuat, 82377 Penzberg (DE); Kuhn, Paul, 82065 Baierbrunn (DE)
(74) Vertreter: Frommberger, Moritz

(57) **Zusammenfassung**

Die Erfindung betrifft ein Verfahren zur trenntechnischen Bearbeitung eines an Wasserstoff, Methan und Kohlenwasserstoffen mit zwei Kohlenstoffatomen reichen Einsatzstroms (a), bei dem aus Fluid des Einsatzstroms (a) ein an Methan und Wasserstoff reicher und an Kohlenwasserstoffen mit zwei Kohlenstoffatomen armer erster Abstrom (b) gewonnen wird. Die Kohlenwasserstoffe mit zwei Kohlenstoffatomen werden aus dem Fluid des Einsatzstroms (a) durch Inkontaktbringen mit Fluid eines an Kohlenwasserstoffen mit zumindest drei Kohlenstoffatomen reichen Waschstroms (c) überwiegend oder vollständig ausgewaschen, wodurch aus dem Fluid des Einsatzstroms (a) der erste Abstrom (b) und ein an Kohlenwasserstoffen mit zwei und mehr Kohlenstoffatomen reicher flüssiger zweiter Abstrom (e) gewonnen werden. Eine entsprechende Anlage (100) ist ebenfalls Gegenstand der Erfindung.

## Beschreibung

Die Erfindung betrifft ein Verfahren zur trenntechnischen Bearbeitung eines an Wasserstoff, Methan und Kohlenwasserstoffen mit zwei Kohlenstoffatomen reichen Einsatzstroms und eine entsprechende Anlage gemäß den Oberbegriffen der unabhängigen Patentansprüche.

### Stand der Technik

Verfahren und Anlagen zum Dampfspalten von Kohlenwasserstoffen sind beispielsweise im Artikel "Ethylene" in Ullmann's Encyclopedia of Industrial Chemistry, Onlineausgabe, 15. April 2007, DOI 10.1002/14356007.a10_045.pub2, beschrieben. Das Dampfspalten (engl. Steam Cracking) wird beispielsweise zur Gewinnung von kurzkettigen Olefinen wie Ethylen und Propylen, Diolefinen wie Butadien oder von Aromaten eingesetzt, ist jedoch nicht hierauf beschränkt.

Durch Dampfspalten wird dabei ein Komponentengemisch erhalten, das zunächst als Rohgas bezeichnet wird. Dieses wird mehreren Aufbereitungsschritten, beispielsweise einer Rohgaswäsche, einer Rohgasverdichtung und einer sogenannten Primärfraktionierung unterworfen. Das auf diese Weise aufbereitete Rohgas wird anschließend einer Trennung zugeführt, die zur Gewinnung von Komponenten oder Komponentengruppen des Rohgases auf Grundlage ihrer Siedepunkte dient. Es kann auch vorgesehen sein, bestimmte Komponenten des Rohgases vor oder innerhalb einer entsprechenden Trennung, beispielsweise durch Hydrieren, umzusetzen.

Eine typische Trennung umfasst mehrere Trennschritte, bei denen jeweils Komponentengruppen gewonnen werden. Beispielsweise sind hierbei "Demethanizer First"-, "Deethanizer First"- oder auch "Depropanizer First"- Verfahren bekannt. Zu Details sei auf die Fachliteratur, beispielsweise den erwähnten Artikel "Ethylene" in Ullmann's Encyclopedia of Industrial Chemistry, verwiesen.

Die vorliegende Erfindung betrifft dabei insbesondere Trennungen, in denen ein an Wasserstoff, Methan und Kohlenwasserstoffen mit zwei Kohlenstoffatomen reiches Komponentengemisch, ein sogenannter C2minus-Strom (siehe unten) gebildet wird, aus welchem die Kohlenwasserstoffe mit zwei Kohlenstoffatomen zunächst in einer gemeinsamen Fraktion gewonnen werden sollen.

Zur Behandlung entsprechender Ströme sind beispielsweise die unten erläuterten Verfahren aus dem Stand der Technik bekannt, die je nach den beim Dampfspalten umgesetzten Verbindungen unterschiedlich ausgestaltet sein können. Diesen Verfahren ist gemeinsam, dass hierbei Temperaturen von -100 °C und weniger zur Abtrennung von Methan und Wasserstoff zum Einsatz kommen müssen, was die Verwendung von Ethylen als Kältemittel erfordert. Gegebenenfalls ist dabei der Einsatz von Turboexpandern zur Erzielung von Temperaturen von -130 °C und weniger erforderlich, insbesondere dann, wenn beim Dampfspalten überwiegend oder ausschließlich gasförmige bzw. ethanreiche Einsätze verwendet werden.

Die Erfindung stellt sich die Aufgabe, entsprechende Verfahren und Anlagen zu vereinfachen und ihren Betrieb effizienter zu gestalten.

### Offenbarung der Erfindung

Diese Aufgabe wird durch ein Verfahren zur trenntechnischen Bearbeitung eines an Wasserstoff, Methan und Kohlenwasserstoffen mit zwei Kohlenstoffatomen reichen Einsatzstroms und eine entsprechende Anlage mit den Merkmalen der unabhängigen Patentansprüche gelöst. Ausgestaltungen sind jeweils Gegenstand der abhängigen Patentansprüche und der nachfolgenden Beschreibung.

Vor der Erläuterung der Merkmale und Vorteile der vorliegenden Erfindung werden deren Grundlagen und die verwendeten Begriffe erläutert.

Gängige Verfahren zur Trennung von Produktströmen aus Verfahren zur Herstellung von Kohlenwasserstoffen umfassen die Bildung einer Reihe von Fraktionen auf Grundlage der unterschiedlichen Siedepunkte der enthaltenen Komponenten. In der Fachwelt werden hierfür Kurzbezeichnungen verwendet, die die Kohlenstoffanzahl der jeweils überwiegend oder ausschließlich enthaltenen Kohlenwasserstoffe angeben. So ist eine "C1-Fraktion" eine Fraktion, die überwiegend oder ausschließlich Methan (und konventionsgemäß unter Umständen auch Wasserstoff, dann auch "C1 minus-Fraktion" genannt) enthält. Eine "C2-Fraktion" enthält hingegen überwiegend oder ausschließlich Ethan, Ethylen und/oder Acetylen. Eine "C3-Fraktion" enthält überwiegend Propan, Propylen, Methylacetylen und/oder Propadien. Eine "C4-Fraktion" enthält überwiegend oder ausschließlich Butan, Buten, Butadien und/oder Butin, wobei die jeweiligen Isomere je nach Quelle der C4-Fraktion in unterschiedlichen Anteilen enthalten sein können. Entsprechendes gilt auch für die "C5-Fraktion" und die höheren Fraktionen. Mehrere solcher Fraktionen können zusammengefasst werden. Beispielsweise enthält eine "C2plus-Fraktion" überwiegend oder ausschließlich Kohlenwasserstoffe mit zwei und mehr und eine "C2minus-Fraktion" überwiegend oder ausschließlich Kohlenwasserstoffe mit einem oder zwei Kohlenstoffatomen. Entsprechende Fraktionen können auch als Kältemittel eingesetzt werden, so beispielsweise die C2- oder C3-Fraktionen. Die durch entsprechende C2- oder C3-Kältemittel bereitstellbaren Temperaturniveaus werden landläufig auch als "C2-Kälte" oder "C3-Kälte" bezeichnet.

Flüssige und gasförmige Ströme können im hier verwendeten Sprachgebrauch reich oder arm an einer oder mehreren Komponenten sein, wobei "reich" für einen Gehalt von wenigstens 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Flüssige und gasförmige Ströme können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem der flüssige oder gasförmige Strom erhalten wurde. Der flüssige oder gasförmige Strom ist "angereichert", wenn dieser zumindest den 1,1-fachen, 1,5-fachen, 2-fachen, 5-fachen, 10-fachen, 100-fachen oder 1.000-fachen Gehalt, "abgereichert", wenn er höchstens den 0,9-fachen, 0,5-fachen, 0,1-fachen, 0,01-fachen oder 0,001-fachen Gehalt einer entsprechenden Komponente, bezogen auf das Ausgangsgemisch, enthält. Ein "überwiegend" eine oder mehrere Komponenten enthaltender Strom enthält diese eine oder mehreren Komponenten zumindest zu 50% oder ist reich an diesen im obigen Sinn.

Ein flüssiger oder gasförmiger Strom ist von einem anderen flüssigen oder gasförmigen Strom (auch als Ausgangsstrom bezeichnet) "abgeleitet", wenn er zumindest einige in dem Ausgangsstrom enthaltene oder aus diesem erhaltene Komponenten aufweist. Ein in diesem Sinne abgeleiteter Strom kann aus dem Ausgangsstrom durch Abtrennen oder Abzweigen eines Teilstroms oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten werden.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Zur Auslegung und spezifischen Ausgestaltung von Destillationssäulen und Absorptionskolonnen, wie sie auch im Rahmen der vorliegenden Anmeldung eingesetzt werden können, sei auf einschlägige Lehrbücher verwiesen (siehe beispielsweise Sattler, K.: Thermische Trennverfahren: Grundlagen, Auslegung, Apparate, 3. Auflage 2001, Weinheim, Wiley-VCH).

Bei einer "Destillationssäule" handelt es sich im hier verwendeten Sprachgebrauch um eine Trenneinheit, die dafür eingerichtet ist, ein gasförmig oder flüssig oder in Form eines Zweiphasengemischs mit flüssigen und gasförmigen Anteilen, ggf. auch im überkritischen Zustand, bereitgestelltes Stoffgemisch zumindest teilweise aufzutrennen, also aus dem Stoffgemisch jeweils Reinstoffe oder Stoffgemische zu erzeugen, die gegenüber dem Stoffgemisch bezüglich zumindest einer Komponente angereichert bzw. abgereichert im oben erläuterten Sinne sind. Destillationssäulen sind aus dem Bereich der Trenntechnik hinlänglich bekannt. Typischerweise sind Destillationssäulen als zylindrische Metallbehälter ausgebildet, die mit Einbauten, beispielsweise Siebböden oder geordneten oder ungeordneten Packungen, ausgerüstet sind. Eine Destillationssäule zeichnet sich unter anderem dadurch aus, dass sich in ihrem unteren Bereich, auch als Sumpf bezeichnet, eine flüssige Fraktion abscheidet. Diese flüssige Fraktion, auch als Sumpfprodukt bezeichnet, wird in einer Destillationssäule mittels eines Sumpfverdampfers erwärmt, so dass kontinuierlich ein Teil des Sumpfprodukts verdampft und in der Destillationssäule gasförmig aufsteigt. Eine Destillationssäule ist ferner typischerweise mit einem sogenannten Kopfkondensator versehen, in den zumindest ein Teil eines sich in einem oberen Bereich der Destillationssäule anreichernden Gasgemischs oder ein entsprechendes Reingas, auch als Kopfprodukt bezeichnet, eingespeist, dort verflüssigt und als flüssiger Rücklauf am Kopf der Destillationssäule aufgegeben wird.

Im Gegensatz zu einer Destillationssäule verfügt eine "Absorptionskolonne" nicht über einen Sumpfverdampfer. Auch Absorptionskolonnen sind aus dem Bereich der Trenntechnik allgemein bekannt. Absorptionskolonnen werden zur Absorption im Phasengegenstrom verwendet und daher auch als Gegenstromkolonnen bezeichnet. Bei der Absorption im Gegenstrom strömt die abgebende Gasphase aufwärts durch eine Absorptionskolonne. Die aufnehmende Lösungsphase fließt, von oben aufgegeben und unten abgezogen, der Gasphase entgegen. In einer entsprechenden Absorptionskolonne sind ebenfalls typischerweise Einbauten vorgesehen, die für einen stufenweisen (Böden, Sprühzonen, rotierende Teller usw.) oder stetigen (regellose Schüttungen von Füllkörpern, Packungen usw.) Phasenkontakt sorgen.

Bei einer Absorption im Gegenstrom, wie er in einer Absorptionskolonne erfolgt, wird ein gasförmiger Strom einem insbesondere fein verteilten flüssigen Strom entgegengeschickt. Der flüssige Strom nimmt dabei Komponenten aus dem gasförmigen Strom auf, die auf diese Weise aus dem gasförmigen Strom ausgewaschen werden. Die Auswaschung kann teilweise oder vollständig sein.

### Vorteile der Erfindung

Die vorliegende Erfindung geht von einem Verfahren zur trenntechnischen Bearbeitung eines an Wasserstoff, Methan und Kohlenwasserstoffen mit zwei Kohlenstoffatomen reichen Einsatzstroms, also eines typischen C2minus-Stroms, aus, bei dem aus Fluid des Einsatzstroms ein an Methan und Wasserstoff reicher und an Kohlenwasserstoffen mit zwei Kohlenstoffatomen armer erster Abstrom gewonnen wird.

Erfindungsgemäß ist vorgesehen, die Kohlenwasserstoffe mit zwei Kohlenstoffatomen aus dem Fluid des Einsatzstroms durch Inkontaktbringen mit Fluid eines an Kohlenwasserstoffen mit zumindest drei, insbesondere zumindest vier, Kohlenstoffatomen reichen Waschstroms überwiegend oder vollständig auszuwaschen, wodurch aus dem Fluid des Einsatzstroms der erste Abstrom und ein an Kohlenstoffen mit zwei und mehr Kohlenstoffatomen reicher flüssiger zweiter Abstrom gewonnen werden. Mit anderen Worten sieht die Erfindung also vor, die Kohlenwasserstoffe mit zwei Kohlenstoffatomen aus dem Einsatzstrom auszuwaschen, wodurch diese in einen Waschabstrom, den zweiten Abstrom, übergehen, in dem diese zusammen mit den als Waschmitteln verwendeten Kohlenwasserstoffen mit drei und mehr oder vier und mehr Kohlenstoffen vorliegen.

Als Waschmittel können im Rahmen der vorliegenden Erfindung beispielsweise Flüssigerdgas (engl. Liquefied Petroleum Gas, LPG) oder auch C4-, C5- oder schwerere Kohlenwasserstoffe und entsprechende Gemische verwendet werden. Besondere Vorteile bieten butanreiche Gemische, insbesondere sogenanntes C4-LPG. Der Waschstrom kann grundsätzlich Kohlenwasserstoffe mit drei, vier, fünf, sechs, sieben, acht, neun und/oder zehn Kohlenstoffatomen und/oder beliebige Gemische in einer Menge von jeweils mehr als 10%, 20%, 30%, 40%, 50%, 60%, 70%, 80% oder 90% aufweisen, weist jedoch insbesondere überwiegend oder ausschließlich Kohlenwasserstoffe mit vier, fünf und sechs, vorzugsweise überwiegend oder ausschließlich Kohlenwasserstoffe mit vier und fünf Kohlenstoffatomen auf.

Wie bereits eingangs erläutert, ist es in herkömmlichen Verfahren zur Bearbeitung entsprechender Einsatzströme erforderlich, ausgesprochen tiefe Temperaturen von bis zu -100 °C und ggf. auch weniger bereitzustellen un d ggf. zusätzlich Turboexpander einzusetzen. Beides schafft Nachteile, weil bei entsprechend tiefen Temperaturen teure, tieftemperaturfeste Materialien verwendet werden müssen bzw. besondere Anforderungen an drehende Maschinen zu stellen sind.

Die Erfindung behebt diesen Nachteil durch das Auswaschen von Kohlenwasserstoffen mit zwei Kohlenstoffatomen aus einem entsprechenden Strom. Sie ersetzt damit die klassischen Tieftemperaturkomponenten in herkömmlichen Anlagen. Der als Einsatzstrom verwendete C2minus-Strom wird dabei absorptiv von C2-Komponenten befreit, die mittels des mehrfach erwähnten Waschmittels ausgewaschen werden.

Bei dem als Einsatzstrom verwendeten C2-Strom kann es sich beispielsweise um ein Kopfprodukt eines Deethanizers oder den Austrittsstrom einer C2-Hydrierung einer entsprechenden Ethylen- bzw. Dampfspaltanlage handeln. Ein entsprechender Einsatzstrom wird zur Verwendung im Rahmen der vorliegenden Erfindung, wie auch nachfolgend noch angegeben, vorteilhafterweise auf ein Temperaturniveau von -30 bis -40 °C, beispielsweise auf -35 bis -37 °C abgekühlt , wozu ein C3-Kältemittel wie Propan und/oder Propylen bei niedrigem Druck, verwendet werden kann.

Die Erfindung schafft den besonderen Vorteil, dass, insbesondere in Kombination mit einem angeschlossenen, bei vergleichsweise hohem Druck betriebenen C2-Splitter lediglich das durch C3-Kältemittel bereitstellbare Kälteniveau erforderlich ist. Auf einen Ethylenkältekreislauf kann daher verzichtet werden. Die Erfindung eignet sich in gleicher Weise für sämtliche Typen von Ethylenanlagen, ist daher, im Gegensatz zu herkömmlichen Verfahren, unabhängig von den verwendeten Einsätzen wie Naphtha, Ethan oder Propan. Aufgrund der wärmeren Prozesstemperatur kann einerseits teures Material eingespart werden, andererseits ergibt sich aber auch eine besonders einfache Prozessführung.

Im Rahmen der vorliegenden Erfindung ist es besonders vorteilhaft, wenn der Waschstrom unter Verwendung von Fluid des zweiten Abstroms gebildet wird, aus dem die Kohlenwasserstoffe mit zwei Kohlenstoffatomen überwiegend oder vollständig entfernt werden. Dies kommt einem "Regenerieren" eines entsprechenden Waschstroms einerseits und andererseits der Gewinnung der erwünschten Komponenten, nämlich der Kohlenwasserstoffe mit zwei Kohlenstoffatomen, gleich. Zur Entfernung der Kohlenwasserstoffe mit zwei Kohlenstoffatomen aus dem zweiten Abstrom wird vorteilhafterweise eine Destillationssäule verwendet, deren Kopfprodukt partiell mit C3-Kälte kondensiert werden kann. Die dabei anfallende Flüssigkeit wird als Rücklauf auf eine entsprechende Destillationssäule zurückgeführt. Die dabei nicht kondensierte Gasphase, d.h. das nicht kondensierte Kopfprodukt vom Kopf dieser Destillationssäule, enthält Kohlenwasserstoffe mit zwei Kohlenstoffatomen und stellt daher das gewünschte Produkt da. Eine entsprechende Destillationssäule wird mit einem Sumpfverdampfer betrieben, in den Wärme beispielsweise durch Niederdruckdampf eingeleitet werden kann.

Vorteilhafterweise wird im Rahmen der vorliegenden Erfindung zur Bildung des Waschstroms das Fluid des zweiten Abstroms abgekühlt, nachdem die Kohlenwasserstoffe mit zwei Kohlenstoffatomen überwiegend oder vollständig aus diesem entfernt wurden. Die Abkühlung des zweiten Abstroms erfolgt dabei typischerweise auf mehreren Stufen, wie nachfolgend erläutert.

Die letzte Abkühlstufe bzw. die letzten Abkühlstufen können dabei das Abkühlen mit einem C3-Kältemittel auf unterschiedlichen Temperaturniveaus umfassen. Diese Temperaturniveaus umfassen typischerweise ein erstes Temperaturniveau bei -15 bis -25 °C, insbesondere bei -20 °C, und/oder ein zweit es Temperaturniveau bei -35 bis -40 °C, insbesondere bei -38 °C. Auf diese Weise kann der regenerierte Waschstrom bei günstigen Temperaturen in eine zum Auswaschen der Kohlenwasserstoffe mit zwei Kohlenstoffatomen verwendete Absorptionskolonne eingespeist werden.

Es ist besonders vorteilhaft, wenn unter Verwendung der überwiegend oder vollständig aus dem zweiten Abstrom entfernten Kohlenwasserstoffe mit zwei Kohlenstoffatomen ein an Kohlenwasserstoffen mit zwei Kohlenstoffatomen reicher dritter Abstrom gebildet wird. Dieser stellt den eigentlichen Produktstrom des erfindungsgemäßen Verfahrens dar und kann anschließend weiter aufgetrennt werden.

Ferner wird vorteilhafterweise im Rahmen des erfindungsgemäßen Verfahrens das Fluid des zweiten Abstroms mit einem oder mehreren, aus Fluid des dritten Abstroms gebildeten Strömen abgekühlt. Der dritte Abstrom wird typischerweise einem sogenannten C2-Splitter zugeführt, in dem in diesem enthaltenes Ethan von in diesem enthaltenem Ethylen getrennt wird. Bei der Bildung des Waschstroms kann daher das Fluid des zweiten Abstroms beispielsweise mit einem Ethan- und/oder Ethylenstrom abgekühlt werden. Dies erlaubt eine günstige Vorkühlung, ehe der regenerierte Waschstrom anschließend der Abkühlung auf die zuvor erläuterten ersten und zweiten Temperaturniveaus zugeführt wird.

Vorteilhafterweise wird im Rahmen der vorliegenden Erfindung zum Auswaschen der Kohlenwasserstoffe mit zwei Kohlenstoffatomen aus dem Fluid des Einsatzstroms eine Absorptionskolonne verwendet, auf die das Fluid des Waschstroms aufgegeben wird. Vorteilhafterweise kommt im Rahmen der vorliegenden Erfindung eine zweiteilige Absorptionskolonne zum Einsatz, bei der ein oberer Teil von einem unteren Teil durch einen Flüssigkeitssperrboden getrennt ist. Eine derartige Absorptionskolonne erlaubt eine besonders vollständige Abtrennung der Kohlenwasserstoffe mit zwei Kohlenstoffatomen. Durch den nachfolgend erläuterten Betrieb einer entsprechenden zweiteiligen Absorptionskolonne ist es möglich, in deren oberen Teil sämtliche Kohlenwasserstoffe mit zwei Kohlenstoffatomen aus der Gasphase des Einsatzstroms zu absorbieren. Weil in dem oberen Teil jedoch auch ein Teil des Methans in die Flüssigphase übergeht, ist in dem unteren Teil der zweiteiligen Absorptionskolonne vorgesehen, das absorbierte Methan aus der Flüssigphase auszustrippen. Hierzu wird vorteilhafterweise ein Gas verwendet, das aus einer Flüssigkeit aus dem oberen Teil der zweiteiligen Absorptionskolonne gebildet wird. Hierzu wird diese Flüssigkeit vorteilhafterweise in einem Aufkocher, d.h. einem hierfür eingerichteten Wärmetauscher, beispielsweise mit warmem Wasser, beispielsweise Quenchwasser aus der Rohgasaufbereitung, oder Dampf erwärmt.

Es ist also besonders vorteilhaft, wenn der Waschstrom auf den oberen Teil der Absorptionskolonne als Rücklauf aufgegeben wird und aus einer in dem oberen Teil der Absorptionskolonne sumpfseitig abgeschiedenen Flüssigkeit ein gasförmiger Strom gebildet wird und dieser in den unteren Teil der Absorptionskolonne eingespeist wird.

Wie bereits mehrfach erläutert, ergeben sich besondere Vorteile, wenn die Absorptionskolonne, zumindest deren oberer Teil, auf einem Temperaturniveau von -30 bis - 40 °C, insbesondere bei ca. -35 °C, betri eben wird. Dies bedeutet, dass sowohl der Waschstrom als auch der Einsatzstrom in die Absorptionskolonne auf einem entsprechenden Temperaturniveau eingespeist werden.

Vorteilhafterweise umfasst das erfindungsgemäße Verfahren ferner, dass aus Fluid des ersten Abstroms ein überwiegend Methan enthaltender Strom und ein überwiegend Wasserstoff enthaltender Strom gebildet werden. Hierzu wird der erste Abstrom durch einen Wärmetauscher geführt und anschließend in einen Abscheidebehälter eingespeist. Aus diesem abgezogene flüssige und gasförmige Ströme stellen die überwiegend Methan beziehungsweise überwiegend Wasserstoff enthaltenden Ströme dar. Diese können entspannt und zur Kühlung des ersten Abstroms im zuvor verwendeten Wärmetauscher eingesetzt werden.

Eine erfindungsgemäße Anlage zur trenntechnischen Bearbeitung eines an Wasserstoff, Methan und Kohlenwasserstoffen mit zwei Kohlenstoffatomen reichen Einsatzstroms ist ebenfalls Gegenstand der vorliegenden Erfindung. Diese weist eine Trenneinrichtung auf, die dafür eingerichtet ist, aus Fluid des Einsatzstroms einen an Methan und Wasserstoff reichen und an Kohlenwasserstoffen mit zwei Kohlenstoffatomen armen ersten Abstrom zu gewinnen. Erfindungsgemäß ist vorgesehen, dass die Trenneinrichtung dafür eingerichtet ist, Kohlenwasserstoffe mit zwei Kohlenstoffatomen aus dem Fluid des Einsatzstroms durch Inkontaktbringen mit Fluid eines an Kohlenwasserstoffen mit zumindest drei Kohlenstoffatomen reichen Waschstroms überwiegend oder vollständig auszuwaschen, wodurch aus dem Fluid des Einsatzstroms der erste Abstrom und ein an Kohlenwasserstoffen mit zwei und mehr Kohlenstoffatomen reicher flüssiger zweiter Abstrom gewonnen werden. Die erfindungsgemäße Anlage profitiert von den Merkmalen und Vorteilen, auf die zuvor ausdrücklich Bezug genommen wurde. Insbesondere ist eine derartige Anlage zur Durchführung eines Verfahrens eingerichtet, wie es zuvor erläutert wurde.

Ausführungsformen der Erfindung werden nachfolgend unter Bezugnahme auf die beigefügten Zeichnungen gegenüber dem Stand der Technik näher erläutert.

Kurze Beschreibung der Zeichnungen
Figur 1 zeigt ein nicht erfindungsgemäßes Verfahren in Form eines schematischen Prozessflussdiagramms.
Figur 2 zeigt ein nicht erfindungsgemäßes Verfahren in Form eines schematischen Prozessflussdiagramms.
Figur 3 veranschaulicht ein Verfahren gemäß einer Ausführungsform der Erfindung in Form eines schematischen Prozessflussdiagramms.

### Ausführliche Beschreibung der Zeichnungen

In den Figuren tragen einander entsprechende Elemente entsprechende Bezugszeichen und werden der Übersichtlichkeit halber nicht wiederholt erläutert.

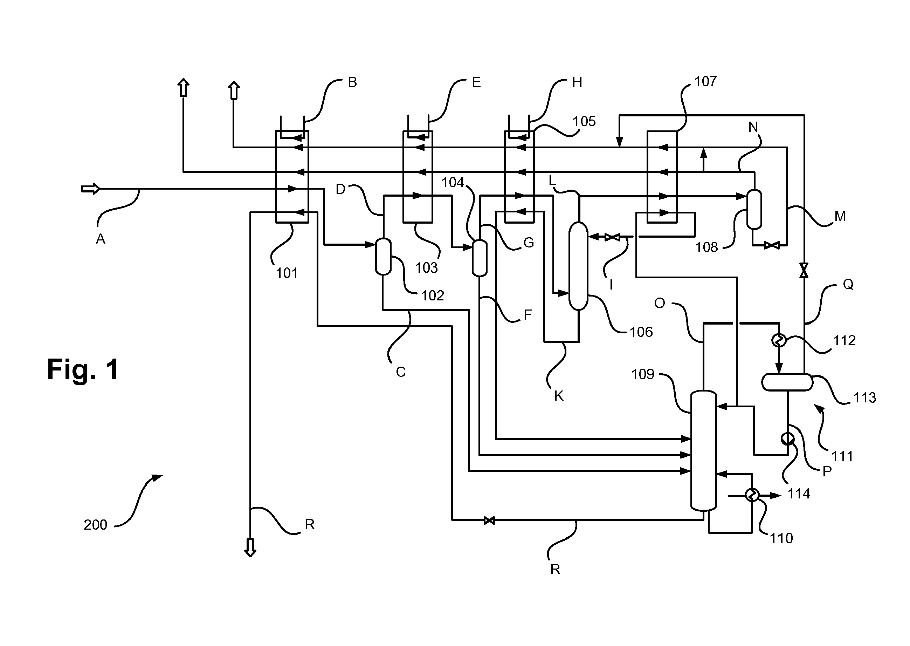

In Figur 1 ist ein nicht erfindungsgemäßes Verfahren zur trenntechnischen Bearbeitung eines an Wasserstoff, Methan und Kohlenwasserstoffen mit zwei Kohlenstoffatomen reichen Einsatzstroms in Form eines schematischen Prozessflussdiagramms veranschaulicht und insgesamt mit 200 bezeichnet. Das Verfahren 200 ist insbesondere zur trenntechnischen Bearbeitung eines an Wasserstoff, Methan und Kohlenwasserstoffen mit zwei Kohlenstoffatomen reichen Einsatzstroms ausgebildet, der aus einem Rohgasstrom eines Dampfspaltverfahrens stammt, bei dem überwiegend oder ausschließlich Flüssige Einsätze, beispielsweise Naphtha, bearbeitet werden, d.h. aus einem Rohgasstrom eines "Flüssigcrackers".

Ein derartiger Einsatzstrom, hier mit A bezeichnet, wird dabei zunächst durch einen ersten Wärmetauscher 101 geführt und unter anderem unter Verwendung eines Kältemittelstroms B, bei welchem es sich beispielsweise um Ethylen auf einem Temperaturniveau von ca. -57 °C handelt, abgekühlt.

Der Strom A wird anschließend in einen ersten Abscheidebehälter 102 überführt, welchem ein flüssiger Strom C und ein gasförmiger Strom D entnommen werden. Der gasförmige Strom D wird durch einen zweiten Wärmetauscher 103 geführt und hier unter anderem mit einem Strom E, bei welchem es sich um Ethylen auf einem Temperaturniveau von ca. -80 °C handelt, weiter abg ekühlt.

Der Strom D wird anschließend in einen weiteren Abscheidebehälter 104 überführt, dem ebenfalls ein flüssiger Strom, hier mit F bezeichnet, und ein gasförmiger Strom, hier mit G bezeichnet, entnommen werden. Der Strom G wird durch einen dritten Wärmetauscher 105 geführt und dort unter anderem mit einem Strom H, bei welchem es sich um Ethylen auf einem Temperaturniveau von ca. -100 °C handelt, abgekühlt.

Der Strom G wird anschließend in eine Absorptionskolonne 106 überführt, welche mit einem methanreichen flüssigem Rücklaufstrom I betrieben wird. Aus dem Sumpf der Absorptionskolonne 106 wird ein flüssiger Strom K abgezogen und in dem Wärmetauscher 105 erwärmt.

Ein gasförmiger Strom L vom Kopf der Absorptionskolonne 106 wird in einem Wärmetauscher 107 weiter abgekühlt und anschließend in einen weiteren Abscheidebehälter 108, den sogenannten Wasserstoffabscheider, überführt. Aus dem Abscheidebehälter 108 flüssig bzw. gasförmig abgezogene Ströme M und N werden in den Wärmetauschern 107, 105, 103 und 101 erwärmt und als methanreiche Fraktion (Strom M) bzw. wasserstoffreiche Fraktion (Strom N) bereitgestellt.

Die bereits erwähnten flüssigen Ströme C, F und K werden in eine Destillationssäule 109 überführt, wobei ihre Einspeisung je nach Zusammensetzung in unterschiedlichen Höhen erfolgt. Die Destillationssäule 109, die auch als Demethanizer bezeichnet wird, wird mit einem Sumpfverdampfer 110 unter Verwendung eines typischen C3-Kältemittels betrieben. Vom Kopf der Destillationssäule 109 wird ein gasförmiger Strom O abgezogen und einem insgesamt mit 111 bezeichneten Kopfkondensator zugeführt. Der Kopfkondensator 111 umfasst dabei einen Wärmetauscher 112, der mit Ethylen auf einem Temperaturniveau von ca. -100 °C als Kält emittel betrieben wird. In einem dem Wärmetauscher 112 nachgeschalteten Abscheidebehälter 113 wird ein flüssiger Strom P erhalten, welcher mittels einer Pumpe 114 zu einem Teil als Rücklauf auf die Destillationssäule 109 und zu einem Teil als Rücklauf auf die Absorptionskolonne 106 in Form des Stroms I aufgegeben wird. Ein nicht verflüssigter Anteil des Stroms O wird in Form des Stroms Q aus dem Abscheidebehälter 113 abgezogen und als methanreicher Strom mit dem Strom M vereinigt.

Durch den erläuterten Betrieb der Destillationssäule 109 kann aus deren Sumpf ein flüssiger Strom R abgezogen werden, der reich an Kohlenwasserstoffen mit zwei Kohlenstoffatomen ist. Dieser wird im dargestellten Beispiel im Wärmetauscher 101 erwärmt und anschließend beispielsweise einer Trennung zur Gewinnung vom Ethan und Ethylen in einem sogenannten C2-Splitter unterworfen.

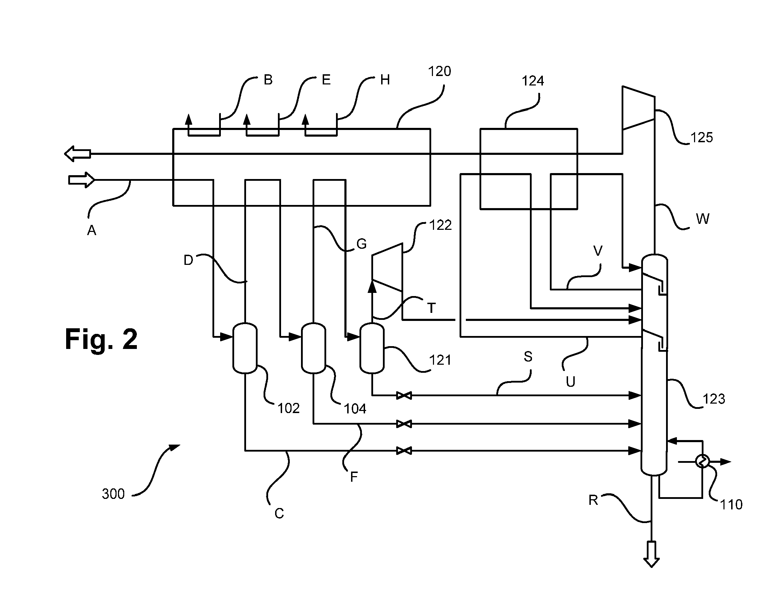

Während sich das in Figur 1 veranschaulichte Verfahren 200 insbesondere für Ströme A eignet, die aus der Dampfspaltung von flüssigen Einsätzen, beispielsweise Naphtha, stammen, ist in Figur 2 ein Verfahren 300 veranschaulicht, das sich insbesondere für die Bearbeitung von Strömen A eignet, die aus einer Dampfspaltung von gasförmigen, insbesondere methanreichen Einsätzen stammen, d.h. aus einem "Gascracker".

Das in Figur 2 veranschaulichte Verfahren, insgesamt mit 300 bezeichnet, verwendet teilweise ähnliche Verfahrensschritte bzw. Vorrichtungen, die daher in Figur 2 identisch wie in Figur 1 bezeichnet sind. Anstelle der drei getrennten Wärmetauscher 101, 103 und 105 der Figur 1 ist hier ein gemeinsamer Wärmetauscherblock 120 veranschaulicht, der jedoch ebenfalls mit entsprechenden Strömen wie in dem Verfahren 200, d.h. mit Ethylen auf einem Temperaturniveau von ca. -57 °C (Strom B), -80 °C (Strom E) und -100°C (Strom H) gekühlt wird. Auch die Gewinnung der flüssigen Ströme C und F in entsprechenden Abscheidebehältern 102 und 104 verläuft grundsätzlich ähnlich, wie in den Verfahren 300. Betont sei jedoch, dass in dem Verfahren 300, das in Figur 2 veranschaulicht ist, grundsätzlich andere Drücke zur Trennung zum Einsatz kommen können.

Anstelle der Absorptionskolonne 106, die in Figur 1 veranschaulicht ist, kommt hier jedoch ein weiterer Abscheidebehälter 121 zum Einsatz, in den der gasförmige Strom G eingespeist wird. Aus dem Abscheidebehälter 121 wird ein flüssiger Strom S und ein gasförmiger Strom T entnommen. Der Strom T wird in einem Turboexpander 122 entspannt und anschließend in eine, hier unterschiedlich zur Destillationssäule 109 des Verfahrens 200, insbesondere mit drei Abschnitten, ausgebildete und daher abweichend bezeichnete Destillationssäule 123 eingespeist. In die Destillationssäule 123, die einen vergleichbar mit dem Sumpfverdampfer 110 der Destillationssäule 109 gemäß Figur 1 aufgebauten und betriebenen Sumpfverdampfer 110 aufweist, werden ferner die zuvor erläuterten flüssigen Ströme C, F und S eingespeist.

Aus einem oberen Teil eines unteren Bereichs der Destillationssäule 123 wird ein gasförmiger Strom U abgezogen, in einem Wärmetauscher 124 abgekühlt und auf einen Mittelteil der Destillationssäule 123 aufgegeben. Aus einem oberen Bereich des Mittelteils der Destillationssäule 123 wird ein weiterer gasförmiger Strom V abgezogen, ebenfalls in dem Wärmetauscher 124 abgekühlt und in einen oberen Teil der Destillationssäule 123 eingespeist. Vom Kopf der Destillationssäule 123 bzw. deren oberen Teils wird ein Strom W gasförmig abgezogen und durch einen Turboexpander 125 entspannt. Die Entspannung des Stroms W in dem Turboexpander 125 liefert die zur Abkühlung der Ströme U und V erforderliche Kälte. Der Strom W trägt ferner zur Abkühlung in dem Wärmetauscher 120 bei. Aus dem Sumpf der Destillationssäule 123 wird schließlich ein Strom R mit vergleichbarer Zusammensetzung wie jener des Stroms R gemäß Figur 1 erhalten und beispielsweise einem C2-Splitter zugeführt.

Wie aus der Zusammenschau der Figuren 1 und 2 ersichtlich, ist in den hier verwendeten Verfahren 200 bzw. 300 die Verwendung von Ethylen als Kältemittel erforderlich, gemäß Verfahren 300 zusätzlich die Verwendung von zwei Turboexpandern.

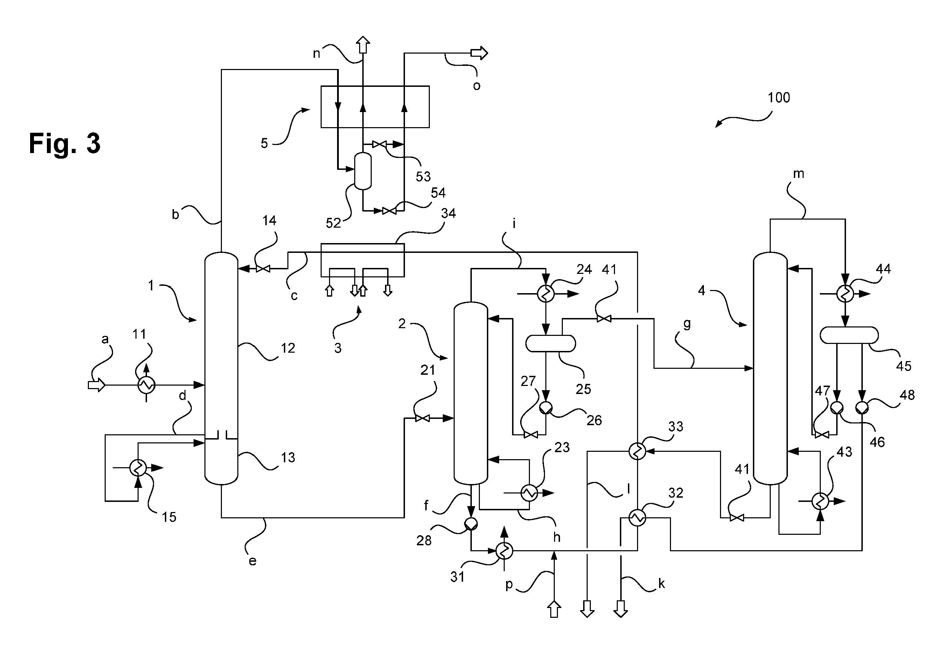

In Figur 3 ist eine Anlage gemäß einer bevorzugten Ausführungsform der Erfindung zur trenntechnischen Bearbeitung eines an Wasserstoff, Methan und Kohlenwasserstoffen mit zwei Kohlenstoffatomen reichen Einsatzstroms in Form eines vereinfachten Prozessflussdiagramms veranschaulicht und insgesamt mit 100 bezeichnet.

In der Anlage 100 wird der erwähnte Einsatzstrom, hier mit a bezeichnet, zunächst durch einen Wärmetauscher 11 geführt und anschließend in den oberen Teil 12 einer Absorptionskolonne 1 überführt. Der Wärmetauscher 11 kann beispielsweise mit einem Kältemittel bei -38 °C betrieben werden und kühlt den Einsatzstrom a auf ca. -35 °C ab. Ein unterer Teil der Absorptionskolonne 1 ist mit 13 bezeichnet. Am Kopf des oberen Teils 12 der Absorptionskolonne 1 wird, wie nachfolgend erläutert, ein aus Fluid des Einsatzstroms a gebildeter, an Methan und Wasserstoff reicher und Kohlenwasserstoffen mit zwei Kohlenstoffatomen armer Abstrom b, hier als "erster" Abstrom bezeichnet, abgezogen, dessen weitere Behandlung unten erläutert wird.

In dem oberen Teil 12 der Absorptionskolonne 1 werden die Kohlenwasserstoffe mit zwei Kohlenstoffatomen aus dem Fluid des Einsatzstroms a durch Inkontaktbringen mit Fluid eines an Kohlenwasserstoffen mit zumindest drei, insbesondere zumindest vier, Kohlenstoffatomen reichen Waschstroms c überwiegend oder vollständig ausgewaschen, wobei das Fluid dieses Waschstroms im dargestellten Beispiel über ein Ventil 14 auf den oberen Teil 12 der Absorptionskolonne 1 aufgegeben wird.

Im Sumpf des oberen Teils 12 der Absorptionskolonne 1 bzw. auf einem Flüssigkeitssperrboden scheidet sich eine Flüssigkeit ab, die neben den Kohlenwasserstoffen mit zwei Kohlenstoffatomen aus dem Fluid des Einsatzstroms a und den höheren Kohlenwasserstoffen des Waschstroms c auch noch Methan enthält. Zum Entfernen des Methans wird diese Flüssigkeit daher im dargestellten Beispiel in Form des Stroms d abgezogen, durch einen Aufkocher 15 geführt, der beispielsweise mit Waschwasser betrieben wird, und in den unteren Teil 13 der Absorptionskolonne 1 eingespeist. Auf diese Weise kann Methan zumindest teilweise aus dem Fluid des Stroms d desorbiert werden, so dass sich im Sumpf der Absorptionskolonne 1 bzw. deren unteren Teils 13 eine Sumpfflüssigkeit abscheidet, die reich an Kohlenwasserstoffen mit zwei Kohlenstoffatomen, jedoch arm an leichteren Kohlenwasserstoffen und Wasserstoff ist.

Die Sumpffraktion wird im dargestellten Beispiel in Form eines Abstroms e, hier als "zweiter" Abstrom bezeichnet, gewonnen und über ein Ventil 21 in eine Destillationssäule 2 überführt. Die Destillationssäule 2 dient zur Auftrennung des zweiten Abstroms e und zur Gewinnung eines an Kohlenwasserstoffen mit zumindest drei Kohlenstoffatomen reichen Stroms f und eines an Kohlenwasserstoffen mit zwei Kohlenstoffatomen reichen Stroms g, hier als "dritter" Abstrom bezeichnet. Zur Gewinnung der genannten Ströme f und g wird aus dem Sumpf der Destillationssäule 2 ein Strom h abgezogen, in einem Wärmetauscher 23, der einen Sumpfverdampfer der Destillationssäule 2 bildet, erwärmt, und zurück in die Destillationssäule 2 geführt. Der Wärmetauscher 23 kann mit Wasser als Heizmedium betrieben werden. Zudem wird der bereits erwähnte, überwiegend Kohlenwasserstoffe mit zumindest drei Kohlenstoffatomen enthaltende Strom f aus der Destillationssäule 2 abgezogen und wie nachfolgend erläutert weiter behandelt.

Vom Kopf der Destillationssäule 2 wird ein gasförmiger Strom i abgezogen und durch einen Wärmetauscher 24 abgekühlt. Der Wärmetauscher 24 kann beispielsweise mit Propan als Kältemittel auf -38 °C betrieben werden. Anschließend wird der Strom i in einen Abscheidebehälter 25 überführt. Vom Sumpf des Abscheidebehälters 25 wird ein nicht näher bezeichneter Strom abgezogen und mittels einer Pumpe 26 über ein Ventil 27 auf den Kopf der Destillationssäule 2 zurückgeführt.

Ein in dem Abscheidebehälter 25 gasförmig verbleibender Teil wird über ein Ventil 41 in eine weitere Destillationssäule 4 als der bereits erwähnte Strom g überführt. Die weitere Destillationssäule 4 dient zur Auftrennung von in dem Strom g, also dem dritten Abstrom, enthaltenen Kohlenwasserstoffen mit zwei Kohlenstoffatomen, insbesondere Ethan und Ethylen, wobei ein ethylenreicher Strom in Form des Stroms k und ein ethanreicher Strom in Form des Stroms I bereitgestellt wird. Zur Bereitstellung des Stroms k wird vom Kopf der Destillationssäule 4 ein Strom m abgezogen und durch einen Wärmetauscher 44, der beispielsweise ebenfalls mit Propan- bzw. C3-Kälte bei -38 °C betrieben wird, gekühlt. Fluid des Stroms m wird anschließend in einen Abscheidebehälter 45 überführt, von dessen Sumpf einerseits ein nicht näher bezeichneter Strom abgezogen und mittels einer Pumpe 46 und eines Ventils 47 zum Kopf der Destillationssäule 42 zurückgeführt wird. Ein weiterer Teil wird in Form des Stroms k abgezogen und mittels einer Pumpe 48 über einen Wärmetauscher 32 aus der Anlage 100 ausgeführt.

Zur Bereitstellung des Strom I wird aus dem Sumpf der Destillationssäule 4, die ferner mit einem Sumpfverdampfer 43 betrieben wird, der Strom I über ein Ventil 41 ausgeleitet und über einen Wärmetauscher 33 aus der Anlage 100 herausgeführt. Der Wärmetauscher 43 des Sumpfverdampfers der Destillationssäule 4 wird beispielsweise mittels druckbeaufschlagtem Propan als Wärmemedium betrieben. Der bereits oben erwähnte Strom f, also ein Strom, der aus dem zweiten Abstrom e gebildet wird und überwiegend oder ausschließlich Kohlenwasserstoffe mit zumindest drei Kohlenstoffatomen enthält, wird aus der Destillationssäule 2 mittels einer Pumpe 28 durch einen Wärmetauscher 31, der beispielsweise mittels Kühlwasser betrieben wird, und anschließend durch die bereits erwähnten Wärmetauscher 32 und 33 geführt. Zuvor wird der Strom f mit einem Frischeinsatzstrom p, der reich an Kohlenwasserstoffen mit zumindest drei Kohlenstoffatomen ist, vereinigt.

Nach der Vereinigung der Ströme f und e und der Abkühlung in den genannten Wärmetauschern 31 bis 33 wird ein entsprechend vereinigter Strom c, der bereits erläuterte Waschstrom, durch einen weiteren Wärmetauscher 34, beispielsweise einen Plattenwärmetauscher, der mit zwei getrennten Propankältemittelkreisläufen auf unterschiedlichen Temperaturniveaus, beispielsweise -38 °C und -20 °C betrieben wird, geführt. Der auf diese Weise auf eine Temperatur von ca. -35 °C abgekühlte Strom c wird über das erläuterte Ventil 14 auf die Absorptionskolonne 1 aufgegeben.

Vom Kopf der Absorptionskolonne 1 bzw. deren oberen Teils wird der bereits erwähnte erste Abstrom b abgezogen und zur Gewinnung eines wasserstoffreichen Stroms n und eines methanreichen Stroms o wie nachfolgend erläutert behandelt.

Der erste Abstrom b wird zunächst durch einen Wärmetauscher 5 geführt und in einen Abscheidebehälter 52 eingespeist. Am Kopf des Abscheidebehälters 52 wird eine wasserstoffreiche Fraktion in Form des erläuterten Stroms n abgezogen, aus dem Sumpf des Abscheidebehälters 52 wird eine überwiegend Methan enthaltende Fraktion über ein Ventil 54 abgezogen. Der Strom o kann durch eine Vereinigung mit zumindest einem Teil des Stroms n bereitgestellt werden, wozu ein Ventil 53 vorgesehen ist. Damit kann eine gewünschte Reinheit der Ströme eingestellt werden.

## Patentansprüche

1. Verfahren zur trenntechnischen Bearbeitung eines an Wasserstoff, Methan und Kohlenwasserstoffen mit zwei Kohlenstoffatomen reichen Einsatzstroms (a), bei dem aus Fluid des Einsatzstroms (a) ein an Methan und Wasserstoff reicher und an Kohlenwasserstoffen mit zwei Kohlenstoffatomen armer erster Abstrom (b) gewonnen wird, **dadurch gekennzeichnet, dass** die Kohlenwasserstoffe mit zwei Kohlenstoffatomen aus dem Fluid des Einsatzstroms (a) durch Inkontaktbringen mit Fluid eines an Kohlenwasserstoffen mit zumindest drei, insbesondere zumindest vier, Kohlenstoffatomen reichen Waschstroms (c) überwiegend oder vollständig ausgewaschen werden, wodurch aus dem Fluid des Einsatzstroms (a) der erste Abstrom (b) und ein an Kohlenwasserstoffen mit zwei und mehr Kohlenstoffatomen reicher flüssiger zweiter Abstrom (e) gewonnen werden.

2. Verfahren nach Anspruch 1, bei dem der Waschstrom (c) unter Verwendung von Fluid des zweiten Abstroms (e) gebildet wird, aus dem die Kohlenwasserstoffe mit zwei Kohlenstoffatomen überwiegend oder vollständig entfernt werden.

3. Verfahren nach Anspruch 2, bei dem der Waschstrom (c) ferner unter Verwendung von Fluid eines an Kohlenwasserstoffen mit zumindest drei Kohlenstoffatomen reichen Frischeinsatzes (p) gebildet wird.

4. Verfahren nach Anspruch 2 oder 3, bei dem zur Bildung des Waschstroms (c) das Fluid des zweiten Abstroms abgekühlt wird, nachdem die Kohlenwasserstoffe mit zwei Kohlenstoffatomen überwiegend oder vollständig entfernt wurden.

5. Verfahren nach Anspruch 4, bei dem das Abkühlen das Abkühlen mit einem C3-Kältemittel auf unterschiedlichen Temperaturniveaus umfasst.

6. Verfahren nach Anspruch 5, bei dem die Temperaturniveaus ein erstes Temperaturniveau bei -15 bis -25 °C, insbesondere - 20 °C, und/oder ein zweites Temperaturniveau bei -35 bis -40 °C, insbesondere -38 °C, umfassen.

7. Verfahren nach einem der Ansprüche 2 bis 6, bei dem unter Verwendung der überwiegend oder vollständig aus dem zweiten Abstrom (e) entfernten Kohlenwasserstoffe mit zwei Kohlenstoffatomen ein an Kohlenwasserstoffen mit zwei Kohlenstoffatomen reicher dritter Abstrom (g) gebildet wird.

8. Verfahren nach Anspruch 7, bei dem bei der Bildung des Waschstroms (c) das Fluid des zweiten Abstroms ferner mit einem oder mehreren, aus Fluid des dritten Abstroms (g) gebildeten Strömen (l, k) abgekühlt wird.

9. Verfahren nach einem der vorstehenden Ansprüche, bei dem zum Auswaschen der Kohlenwasserstoffe mit zwei Kohlenstoffatomen aus dem Fluid des Einsatzstroms (a) eine Absorptionskolonne (1) verwendet wird, auf die das Fluid des Waschstroms (c) aufgegeben wird.

10. Verfahren nach Anspruch 9, bei dem eine zweiteilige Absorptionskolonne (1) verwendet wird, bei der ein oberer Teil (12) von einem unteren Teil (13) durch einen Flüssigkeitssperrboden getrennt ist.

11. Verfahren nach Anspruch 10, bei dem aus in dem oberen Teil (12) der Absorptionskolonne (1) abgeschiedener Flüssigkeit ein gasförmiger Strom (d) gebildet und in den unteren Teil (13) der Absorptionskolonne (1) eingespeist wird.

12. Verfahren nach Anspruch 9 oder 10, bei dem der Waschstrom (c) auf den oberen Teil (12) der Absorptionskolonne (1) als Rücklauf aufgegeben wird.

13. Verfahren nach einem der Ansprüche 9 bis 11, bei dem der Waschstrom (c) und der Einsatzstrom (a) in die Absorptionskolonne (1) auf einem Temperaturniveau von -30 bis -40 °C, insbesondere -35 °C, eingespeis t wird.

14. Verfahren nach einem der vorstehenden Ansprüche, bei dem aus Fluid des ersten Abstroms (b) ein überwiegend Methan enthaltender Strom (o) und ein überwiegend Wasserstoff enthaltender Strom (n) gebildet wird.

15. Anlage (100) zur trenntechnischen Bearbeitung eines an Wasserstoff, Methan und Kohlenwasserstoffen mit zwei Kohlenstoffatomen reichen Einsatzstroms (a), mit einer Trenneinrichtung, die dafür eingerichtet ist, aus Fluid des Einsatzstroms (a) einen an Methan und Wasserstoff reichen und an Kohlenwasserstoffen mit zwei Kohlenstoffatomen armen ersten Abstrom (b) zu gewinnen, **dadurch gekennzeichnet, dass** die Trenneinrichtung dazu eingerichtet ist, Kohlenwasserstoffe mit zwei Kohlenstoffatomen aus dem Fluid des Einsatzstroms
(a) durch Inkontaktbringen mit Fluid eines an Kohlenwasserstoffen mit zumindest drei Kohlenstoffatomen reichen Waschstroms (c) überwiegend oder vollständig auszuwaschen, wodurch aus dem Fluid des Einsatzstroms (a) der erste Abstrom
(b) und ein an Kohlenwasserstoffen mit zwei und mehr Kohlenstoffatomen reicher flüssiger zweiter Abstrom (e) gewonnen werden.
